(19) Europäisches Patentamt
European Patent Office
Office européen des brevets

(11) Publication number: 0 253 662 B1

# (12) EUROPEAN PATENT SPECIFICATION

(45) Date of publication of the patent specification: 14.11.90

(51) Int. Cl.⁵: **A61K 31/54**, A61K 47/00

(21) Application number: 87306297.0

(22) Date of filing: 16.07.87

(54) **Pharmaceutical compositions.**

(30) Priority: 17.07.86 GB 8617482

(43) Date of publication of application: 20.01.88 Bulletin 88/3

(45) Publication of the grant of the patent: 14.11.90 Bulletin 90/46

(84) Designated Contracting States: AT BE CH DE ES FR GB GR IT LI LU NL SE

(56) References cited:
EP-A- 0 139 535
EP-A- 0 162 705
FR-A- 2 309 231

CHEMICAL ABSTRACTS, vol. 104, 3rd February 1986, page 19, abstract no. 28429m, Columbus, Ohio, US; M. HANDROCK et al.: "Comparative study of the local ototoxicity from taurolin and other antibacterially active substances", & TAUROLIN 1985, 120-30 using YPEB" & J. ELECTROCHEM.
SOC. 1981, 128(11), 2453-60reflaxicon" 000

(73) Proprietor: Ed. Geistlich Söhne A.G. für Chemische Industrie, CH-6110 Wolhusen Lucerne(CH)

(72) Inventor: Pfirrmann, Rolf Wilhelm, Schadrudistrasse 27, Ch-6006 Lucerne(CH)

(74) Representative: Holmes, Michael John et al, Frank B. Dehn & Co. Imperial House 15-19 Kingsway, London WC2B 6UZ(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to sterile aqueous solutions of taurolidine and/or taurultam for parenteral administration.

The antibacterial compounds taurolidine and taurultam have the following formulae:

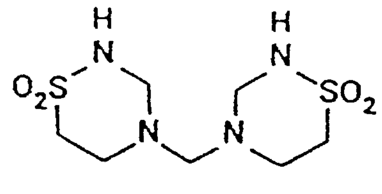

TAUROLIDINE

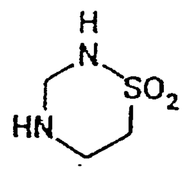

TAURULTAM

These compounds are condensation products of formaldehyde with taurinamide and are active not only against both gram-positive and gram-negative bacteria but also against exotoxins and endotoxins produced by these organisms.

The mode of action of taurolidine has been shown to include the transfer of methylol groups to hydroxyl or amino groups present on the above toxins or on the mureine of the bacterial cell walls. In solution, taurolidine exists in equilibrium with taurultam and N-methylol taurultam, taurolidine being greatly predominant. Taurultam is itself in equilibrium with methylol taurinamide, the equilibrium being greatly in favour of taurultam. When the above methylol derivatives, methylol taurultam and methylol taurinamide, contact the toxins or bacteria, methylol groups are transferred. Methylol taurultam is thereby converted to taurultam, while methylol taurinamide is converted to taurine, a naturally occurring aminosulphonic acid which is extremely well tolerated in the human body. It will thus be appreciated that taurolidine and taurultam act in essentially the same way and produce the same final products.

Taurolidine and taurultam have previously been proposed primarily for use in maintaining sterility in surgical operations where gross infection with bacteria and bacterial toxins was likely, for example in cases of peritonitis. More recently, however, it has been found that bacterial toxaemia can advantageously be treated parenterally. Bacterial endotoxins and exotoxins are primarily responsible for many of the most serious pathological effects of bacterial infections. When such toxins are present at above certain concentrations, the patient can suffer septic shock. It is often found that use of antibiotics is contra-indicated in the case of some gram-negative bacterial infections due to the release of large quantities of endotoxins when the cell walls of gram-negative bacteria are attacked by the antibiotic. It is thus particularly useful in such cases to be able to administer taurolidine and taurultam parenterally, for example intravenously both to combat infections and to deal with septic shock.

However, the water-solubility of both compounds is relatively low, namely l g/litre in the case of taurolidine and 8 g/litre in the case of taurultam. Relatively large quantities of the compounds are required where toxaemia is well established and even where the compounds are administered in large volumes by intravenous infusion, low solubility can limit their use. In particular, during treatment of patients suffering from septic shock and consequent renal insufficiency, administration of significant quantities of liquid (after an initial period of volume compensation) is contra-indicated and the intravenous infusion solution should be as concentrated as possible. Where glucose is present, this is also beneficial in such cases in order to support the brain cells.

Taurolidine has previously been formulated in aqueous solution at concentrations up to 2% by weight by incorporating polyvinyl pyrrolidone as a crystallisation inhibitor. At higher concentrations of taurolidine, however, crystallisation can occur, so rendering the solutions unuseable. Our European Patent Application 139 535 describes such solutions containing polyvinylpyrrolidone and gives the concentration range for the antibacterial as 0.5 to 5%. However this range applies to a group of related compounds and lower levels are recommended for taurolidine.

In view of the mode of action of taurolidine mentioned above, it was not initially thought suitable to use compounds containing hydroxyl groups to increase the solubility or inhibit the crystallisation of the compound. In the case of bacteria and their endo- and exotoxins, it has been found that after the methylol transfer as set out above, there is a further irreversible step involving dehydration. Thus, in the case of bacterial endotoxins, which are lypopolysaccharides, it is found that an irreversible cross-linking reaction takes place which prevents the endotoxin from exerting its lethal effect. Similarly, in the case of bacterial exotoxins, which are proteins or polypeptides and do not contain lypopolysaccharide material of the kind found in the endotoxins, the detoxification reaction is found to be irreversible. However, our investigations have shown that the transfer of methylol groups by the mechanism set out above is reversible in the case of many hydroxyl or amino compounds, so that an equilibrium is established which does not significantly interfere with the availability of taurolidine.

We have now found that surprisingly polyols such as sugars and sugar alcohols can be used to maintain relatively high concentrations of taurolidine and/or taurultam in aqueous solution without significantly effecting the antibacterial and antitoxin activity.

French Patent Application 7 612 644 discloses the use of taurolidine, taurultam and related compounds in dental products including aqueous mouth rinses, which may contain glycerol, sorbitol or xylitol, although the purpose of these is not stated. There is no reference to solubilisation or to the quantities of such polyols to be used. The only mouth rinse exemplified contains perfume, a wetting agent and ethanol and could not be administered parenterally.

When 2% aqueous taurolidine (containing polyvinylpyrrolidone to aid solubility) is administered intravenously it has sometimes been observed that at high rates of infusion, short duration vagotonic side effects occur, such as meiosis, lachrymation, salivation, bradycardia and excitation. We have found that when taurolidine is administered in glucose solution rather than polyvinylpyrrolidone, such vagotonic effects are not observed. This is a surprising effect of considerable value in ensuring that taurolidine can be given intravenously at a rapid rate of infusion, for example in cases of endotoxin shock. In general, the overall intravenous tolerance of taurolidine is increased by the presence of glucose. The same advantages apply also to taurultam.

Furthermore, the beneficial effect observed with glucose can also be obtained using similar polyhydroxylic compounds such as glycerol, other sugars and also sugar alcohols.

Glycerol, sugars and sugar alcohols are commonly administered parenterally, e.g. intravenously, as components of nutritional solutions, which may additionally contain amino acids and trace elements. However, such solutions are often pyrogenic due to bacterial infection during formulation, which is extremely difficult to avoid. The pyrogens are not, of course, removed when the solutions are finally sterilised. We have found that taurolidine and taurultam are capable of detoxifying such pyrogens. Furthermore, although such nutritional solutions are sterile when infusion is started, there is inevitably transmission of infection from the patient to the solution via the intubation. We have found that incorporation of taurolidine and/or taurultam into such nutritional solutions at anti-bacterial concentrations has the added benefit of maintaining sterility during infusion.

Polyhydroxy compounds are difficult to sterilise by autoclaving due to side reactions which may occur. By including taurolidine or taurultam in bacterial quantities we have found it possible to sterilise the solutions by filtration procedures only.

Further, where amino acids are included in such nutritional solutions, the Maillard reaction can take place. We have found that the presence of taurolidine or taurultam prevents or inhibits this reaction.

According to the present invention, therefore, we provide sterile aqueous solutions for parenteral administration containing a bacterially effective concentration of taurolidine and/or taurultam together with a parenterally acceptable polyol, such as a glycerol or sugar or sugar alcohol.

Suitable polyols for inclusion in the solutions of the invention include carbohydrates, e.g. hexoses such as glucose and fructose, (or mixtures of these such as invert sugar), pentoses such as xylose or polysaccharides such as dextran or hydrolysed starch; glycerol and sugar alcohols such as sorbitol, mannitol or xylitol.

Where the solutions are for use in intravenous infusion, to combat bacterial infections and/or toxic shock it is particularly useful if the polyol can be metabolised. For this reason, glucose and fructose are particularly useful and also sorbitol and xylitol. Glucose is advantageous in that it is particularly readily metabolised and, indeed, can provide a valuable nutritional element. On the other hand, fructose may be advantageous where a patient cannot tolerate large quantities of glucose, for example in cases of diabetes.

The concentration of taurolidine in the solution is preferably in the range 1 to 5%, advantageously 2 to 3%, by weight. The concentration of taurultam is preferably in the range 1 to 7.5%, advantageously 3 to 5%, by weight.

The concentration of the polyol can usefully be in the range 3-40% by weight, In the case of glucose, the concentration is preferably in the range 10-30% by weight, preferably 20%.

Thus, particularly preferred formulations according to the invention are aqueous solutions of taurolidine at a concentration in the range 2-4% by weight containing glucose at a concentration of 10-40% by weight. Formulations containing 3-4% by weight of taurolidine and 15-25% by weight of glucose are especially preferred.

Although the solutions according to the invention are surprisingly stable at room temperature, there is some evidence that reaction can occur at elevated temperatures, for example those used in sterilisation by autoclaving. Consequently, as indicated above, it is preferred that the formulations are prepared by dissolving the taurolidine or taurultam in an aqueous solution of the polyol, which may optionally previously be sterilised, for example by autoclaving, and to complete sterilisation by filtration. If autoclaving is used to sterilise solutions containg a polyol (and optionally taurolidine or taurultam) it is preferable that this be done rapidly, in the substantial absence of oxygen. Thus, the vessel containing the solution can be evacuated and the space above the liquid purged with an inert gas such as nitrogen prior to sealing,

so as to provide a very low partial pressure of oxygen. Furthermore, the presence of heavy metals should be avoided.

Although polyols and, indeed amino acid solutions, are most stable at slightly acid pH, it is preferred that the solutions for infusion should be at about neutral pH.

The principal adverse reaction is the conversion of sugars such as glucose or fructose to aldehydes such as hydroxymethylfurfural and ultimately to acids. This reaction has been shown to be catalysed by aspartic acid, which will sometimes be a component of nutritional solutions containing amino acids. However, if the above precautions are taken, there is no difficulty in preparing the solutions of the invention in sterile form at an acceptable level of titratable acidity (e.g. 0.1 to 0.5, equiv. /litre). As indicated above, it is also possibe to sterilise the solutions by filtration.

The solution will normally be at physiological pH. If necessary, the pH can be adjusted by the addition of an acid or a base. While mineral acids such as hydochloric acid can be used, it is preferred to use a metabolisable acid such as acetic, malic or lactic acid, which does not tend to cause acidosis. The pH can also be adjusted by electrolysis. The relatively high concentrations of sugar or sugar alcohol render the solutions hypertonic but this is physiologically acceptable and, indeed, not uncommon where glucose is used in intravenous alimentation.

Where taurolidine is administered by intravenous infusion in order to treat septic shock, a suitable dose will be 20-30 g taurolidine over a 24 hour period in the case of a 70 kg adult human patient. This will be administered conventionally by catheter. Where the solutions are intended for administration of taurolidine or taurultam as such, the polyol will conveniently be glucose and in general, no other components will be present.

Where the solutions are intended for other forms of infusion therapy and taurolidin or taurultam are added to maintain sterility, a wide range of other components are possible.

For therapy of metabolic acidosis, such other components will include buffer salts such as sodium acetate, sodium carbonate and/or hydrogen carbonate, sodium malate or trometamol. The polyol may, for example, be sorbitol.

For osmotherapy, for example, for treatment of cerebral oedema, the polyol is advantageously a combination of glycerol and glucose and an electrolyte such as sodium chloride and/or acetate may be present. Mannitol may also be used as polyol. Where an isotonic, iso-ionic electrolyte solution is required, the electrolyes may be such as to provide sodium, potassium, calcium, magnesium ions in their physiological proportions together with anions such as chloride, phosphate, glycerophosphate and/or acetate ions. The polyol may conveniently be glucose or fructose. Such solutions may alternatively be enriched in one or more cations, e.g. potassium and magnesium.

Where the solutions are intended for parenteral nutrition, e.g. peripheral venous nutrition, amino acids will be present as well as trace elements and vitamins. The amino acids will generally comprise L-isoleucine, L-leucine, L-lysine, L-methionine, L-phenylalanine, L-threonine, L-tryptphane, L-valine, L-arginine, L-histidine, L-alanine, L-glutamic acid L-proline and glycine. The proportions of the amino acids will generally be those conventional in parenteral nutrition. The vitamins which may be present may include pyridoxine (hydrochloride), inositol, riboflavin (5[1]-phosphate sodium salt) and nicotinamide. Electrolytes where present may include sources of sodium, potassium, calcium and/or magnesium with acetate, malate or chloride ions.

Solutions for plasma replacement (e.g. in treatment of hypovolumic shock, burns, thrombosis etc) may include, for example, dextran or hydroxymethyl starch as plasma extender together with an electolyte such as sodium chloride and/or a further polyol such as glucose.

The following Examples are given by way of illustration only:

Example I

50.0 g Glucose were dissolved in 495 ml distilled water and the pH adjusted to 6.6 with N-sodium hydroxide. This solution was autoclaved and I0.0 g taurolidine were then added. After dissolution of the taurolidine, the volume was made up to 500 ml with sterile distilled water and sterile-filtered prior to sealing in a 500 ml flask.

Example 2

I00.0 g Glucose were dissolved in 495 ml distilled water and the pH adjusted to 6.6 with N-sodium hydroxide. This solution was autoclaved and I0.0 g taurolidine were then added. After dissolution of the taurolidine, the volume was made up to 500 ml with sterile distilled water and sterile-filtered prior to sealing in a 500 ml flask.

Example 3

I00.0 g Glucose were dissolved in 495 ml distilled water and the pH adjusted to 6.6 with N-sodium hydroxide. This solution was autoclaved and 20.0 g taurolidine were then added. After dissolution of the

taurolidine, the volume was made up to 500 ml with sterile distilled water and sterile-filtered prior to sealing in a 500 ml flask.

Example 4 : Solution for therapy of metabolic acidosis

l000ml infusion solution contains:
Sodium acetate 8.2g
Sodium hydrogen carbonate 4.2g
Sodium L-malate 6.2g
Trometamol (THAM) 4.0g
Sorbitol 50.0g
Taurolidine 30.0g
in water for injection

Example 5 : Solution for osmotherapy

l litre contains:
Glycerol l00.0g
Glucose monohydrate for injection 27.5g
Sodium chloride 25.0g
Taurolidine 30.0g
in water for injection

Example 6: Isotonic and isoionic electrolyte solution

1 litre contains:
$Na^-$ 3.151g
$K^-$ 0.156g
$Ca^{--}$ 0.066g
$Mg^{--}$ 0.030g
$Cl^-$ 3.900g
Acetate 2.173g
Glucose Monohydrate for injection 55.0g
Taurolidine 20.0g in water for injection

The taurolidine content may alternatively be 30.0g. The 20.0g taurolidine may also be replaced by 30.0g taurultam.

Example 7: Isotonic and isoionic electrolytic solution

1 litre contains:
$Na^-$ 3.151g
$K^-$ 0.156g
$Ca^{--}$ 0.066g
$Mg^{--}$ 0.030g
$Cl^-$ 3.900g
Acetate 2.173g
Fructose 50.0g
Taurolidine 20.0g in water for injection

The taurolidine content may alternatively be 30.0g. The 20.0g taurolidine may also be replaced by 30.0g taurultam.

Example 8 : Isotonic and isoionic electrolytic solution with increased potassium content.

l litre contains
$Na^+$3.l5lg
$K^+$ 0.l56g
$Ca^{++}$ 0.066g
$Mg^{++}$ 0.030g
$Cl^-$ 3.900g
Acetate 2.820g
Fructose 50.0 g
Taurolidine 20.0 g
in water for injection

The taurolidine content may alternatively be 30.0g
The 20.0g taurolidine may also be replaced by 30.0g taurultam.

Example 9 : Electrolyte solution

I litre contains:
$Na^+$I,I29g $K^+$0.973g
$Mg^{++}$0.06Ig
$Cl^-$I.74Ig
$H_2PO_4$0.960g
LactoseI.78Ig
Glucose Monohydrate for injection55.0 g
Taurolidine20.0 g
in water for injection
The taurolidine content may alternatively be 30.0g
The 20.0g taurolidine may also be replaced by 30.0g taurultam.
The potassium content may be varied between 0.05 and 24.9 millimol.

Example I0 : Isotonic sodium chloride solution containing hydroxyethyl-starch

I litre contains
0-(2-Hydroxy-ethyl)-amylopectin hydrolysateI00.00g
(Hydroxyethyl starch)or 60.00g
(Substitution grade 0.40-0.50) (Average molecular weight: 200,000) Sodium chloride9.00g
($Na^+$:I54mmol, $Cl^-$:I54mmol) Glucose monohydrate for injection55.0 g
Taurolidine20.0 g
in water for injection
The taurolidine content may alternatively be 30.0g
The 20.0g taurolidine may also be replaced by 30.0g taurultam.

Example II : Ringer solution

I Litre contains:
$Na^+$I.I29g
$K^+$0.0522g
$Ca^{++}$0.030g
$Cl^-$I.840g
Glucose monohydrate for injection36.6 g
Taurolidine20.0 g
in water for injection
The taurolidine content may alternatively be 30.0g
The 20.0g taurolidine may also be replaced by 30.0g taurultam.

Example I2 : Solution for osmotherapy

I Litre contains:
$Na^+$I.379g
$Cl^-$I.595g
Acetate0.886g
Ethoxy-azorutoside0.200g
Sorbitol400.0 g
Taurolidine20.0 g
in water for injection
The taurolidine content may alternatively be 30.0g
The 20.0g taurolidine may also be replaced by 30.0g taurultam.

Example I3 : Solution for osmotherapy

I Litre contains:
Mannitol200.0 g
Taurolidine20.0 g
in water for injection
The taurolidine content may alternatively be 30.0g
The 20.0g taurolidine may also be replaced by 30.0g taurultam.

Example l4 : Solution with invert sugar

l Litre contains:
Sodium chloride2.483g
Sodium acetate $3H_2O$3.742g
Potassium chloride0.373g
Calcium chloride0.l53g
Glucose Monohydrate for injectionl4.90 g
Fructosel3.l3 g
Taurolidine20.0 g
in water for injection
The taurolidine content may alternatively be 30.0g
The 20.0g taurolidine may also be replaced by 30.0g taurultam.

Example l5 : Electrolyte solution with l0 mmol potassium

l Litre contains:
Sodium chloride4.968g
Sodium acetate7.485g
Potassium chloride0.746g
Calcium chloride0.368g
Magnesium chloride0.305g
Taurolidine20.0 g
Glucose55.0 g
in water for injection
The taurolidine content may alternatively be 30.0g
The 20.0g taurolidine may also be replaced by 30.0g taurultam.

Example l6 : Potassium-magnesium rich infusion solution

l Litre contains:
Xylitol70.0 g
Glucose Monohydrate for injection33.0 g
Malic acid2.5 g
Potassium chloride3.75 g
Magnesium chloridel.0l5g
Magnesium acetate3.22 g
Tripotassium phosphate2.l23g
Taurolidine20.0 g
in water for injection
The taurolidine content may alternatively be 30.0g
The 20.0g taurolidine may also be replaced by 30.0g taurultam.

Example l7 : Electrolyte solution

l Litre contains
Sodium chloride8.l82g
Potassium chloride0.373g
Calcium chloride0.368g
Magnesium chloride0.305g
Fructosel00.0 g
Taurolidine20.0 g
in water for injection
The taurolidine content may alternatively be 30.0g
The 20.0g taurolidine may also be replaced by 30.0g taurultam.

Example 18 : Fully balanced L-amino acid solutions

| | | |
|---|---|---|
| 1 Litre contains: | | |
| Amino acid content | 5% | 10% |
| Amino acids | | |
| L-Isoleucine | 1.55g | 3.10g |
| L-leucine | 2.20g | 4.40g |
| L-Lysine monohydrochloride | 2.50g | 5.00g |
| L-Methionine | 2.10g | 4.20g |
| L-Phenylalanine | 2.20 | 4.40g |
| L-Threonine | 1.00g | 2.00g |
| L-Tryptophane | 0.45g | 0.90g |
| L-Valine | 1.50g | 3.00g |
| L-Alanine | 6.00g | 12.00g |
| L-Arginine | 4.00g | 8.00g |
| L-Glutamic acid | 9.00g | 18.00g |
| Glycine | 10.00g | 20.00g |
| L-Histidine | 1.00g | 2.00g |
| L-Proline | 7.00g | 14.00g |
| Polyols: | | |
| Sorbitol | 50.00g | 50.00g |
| Xylitol | 50.00g | 50.00g |
| Ethanol | 50.00g | - |
| Vitamins: | | |
| Ascorbic acid | 0.40g | 0.40g |
| Inositol | 0.50g | 0.50g |
| Nicotinamide | 0.06g | 0.06g |
| Pyrodoxine hydrochloride(B) | 0.04g | 0.04g |
| Riboflavin-5'-phosphate sodium | | |
| Electrolytes: | | |
| Potassium hydroxide | 1.68g | 1.68g |
| Magnesium acetate | 1.07g | 1.07g |
| Sodium hydroxide | 1.60g | 1.60g |
| L-Malic acid | 2.01g | 0.67g |
| Taurolidine | 20.00g | 20.00g |

The taurolidine content may alternatively be 30.0g
The 20.0g taurolidine may also be replaced by 30.0g taurultam.

Example I9 : Complete solution for peripheral-venous nutrition

I Litre contains:
L-Isoleucine0.98 g
L-leucine2.33 g
L-Lysine monohydrochloride2.2I g
L-MethionineI.26 g
L-PhenylalanineI.I6 g
L-ThreonineI.26 g
L-Tryptophane0.49 g
L-ValineI.08 g
Arginine3.26 g
L-Histidine monohydrochlorideI.09 g
Acetylcysteine0.26 g
Glycine3.64 g
L-Alanine6.06 g
L-(+)Glutamic acid2.0I g
L-Proline3.26 g
L-Serine3.26 g
N-Acetyl-L-tyrosine0.23 g
Sodium L-hydrogen glutamate $H_2O$3.98 g
Sodium chlorideI.I69g
Potassium chloride2.237g
Magnesium acetate 4 $H_2O$I.072g
Calcium chloride 2 $H_2O$0.368g
Glycerol-I(2)-dihydrogenphosphate mixed with its disodium salt (30/70% W/W)5 $H_2O$3.06I g
Sorbitol30.0 g
Xylitol30.0 g
Taurolidine20.0 g
in water for injection
The taurolidine content may alternatively be 30.0g
The 20.0g taurolidine may also be replaced by 30.0g taurultam.

Example 20: Electrolyte-free peripheral nutrition solution

I Litre contains:
L-Isoleucine7.52g
L-leucineII.38g
L-Lysine9.63g
L-Methionine6.59g
L-Phenylalanine7.76g
L-Threonine6.78g
L-Tryptophane2.9Ig
L-Valine9.53g
L-Histidine4.90g
Sorbitol25.00g
Xylitol25.00g
Taurolidine20.00g
in water for injection
The taurolidine content may alternatively be 30.0g
The 20.0g taurolidine may also be replaced by 30.0g taurultam.

Example 2I: Dextran plasma replacement solutions

I Litre contains:

(a) Dextran(MW 40,000)I00g
NaCl9g
Taurolidine20g
in water for injection
(b) Dextran(MW 40,000)I00g
Glucose50g

Taurolidine20g
in water for injection
(c) Dextran (MW 40,000)60g
NaCl9g
Taurolidine20g
in water for injection
(d) Dextran(MW 70,000)60g
Glucose50g
Taurolidine20g
in water for injection
(e) Dextran(MW 70,000)30g
Taurolidine20g
in Ringer-lactate
($Na^+$130m/mol
$K^+$5.4m/mol
$Ca^{++}$0.9m/mol
$Mg^{++}$1.0m/mol
$Cl^-$27m/mol)

The taurolidine content may alternatively be 30.0g
The 20.0g taurolidine may also be replaced by 30.0g taurultam.

## Claims

1. A sterile aqueous solution for parenteral administration containing a bactericidally effective concentration of taurolidine and/or taurultam together with a parenterally acceptable polyol.
2. An aqueous solution as claimed in claim 1 wherein the polyol is glycerol, a sugar or a sugar alcohol.
3. An aqueous solution as claimed in claim 2 wherein the sugar alcohol is sorbitol or xylitol.
4. An aqueous solution as claimed in claim 1 wherein the polyol is glucose.
5. An aqueous solution as claimed in claim 1 wherein the polyol is fructose.
6. An aqueous solution as claimed in claim 1 wherein the concentration of taurolidine is in the range 1 to 5% by weight and/or the concentration of taurultam is in the range 1 to 7.5% by weight.
7. An aqueous solution as claimed in claim 6 wherein the concentration of taurolidine is in the range 2 to 3% by weight and/or the concentration of taurultam is in the range 3 to 5%.
8. An aqueous solution as claimed in claim 6 comprising taurolidine at a concentration in the range 3 to 4% by weight and glucose at a concentration in the range 15 to 25% by weight.
9. An aqueous solution as claimed in claim 1 further comprising an electrolyte and/or one or more amino acids and/or trace elements and vitamins.
10. A process for the preparation of an aqueous solution as claimed in claim 1 wherein the taurolidine and/or taurultam are dissolved in a sterile aqueous solution of the polyol.
11. Use of taurolidine and/or taurultam together with a parenterally acceptable polyol in the manufacture of a sterile aqueous solution for parenteral nutrition or for the parenteral treatment or prophylaxis of infections.
12. A method of solubilising taurolidine and/or taurultam in a sterile aqueous solution for parenteral administration which comprises the addition thereto of an effective amount of a parenterally acceptable polyol.
13. A method of solubilising taurolidine as claimed in claim 12 wherein the polyol is glucose.

## Patentansprüche

1. Sterile wässrige Lösung zur parentalen Verabreichung, enthaltend eine bakterizid wirksame Konzentration von Taurolidin und/oder Taurultam zusammen mit einem parenteral annehmbaren Polyol.
2. Wässrige Lösung gemäss Anspruch 1, dadurch gekennzeichnet, dass das Polyol Glyzerin, ein Zucker oder ein Zuckeralkohol ist.
3. Wässrige Lösung gemäss Anspruch 2, dadurch gekennzeichnet, dass der Zuckeralkohol Sorbit oder Xylit ist.
4. Wässrige Lösung gemäss Anspruch 1, dadurch gekennzeichnet, dass das Polyol Glukose ist.
5. Wässrige Lösung gemäss Anspruch 1, dadurch gekennzeichnet, dass das Polyol Fruktose ist.
6. Wässrige Lösung gemäss Anspruch 1, dadurch gekennzeichnet, dass die Konzentration des Taurolidins im Bereich von 1 bis 5 Gew.-% und/oder die Konzentration von Taurultam im Bereich von 1 bis 7,5 Gew.-% liegt.
7. Wässrige Lösung gemäss Anspruch 6, dadurch gekennzeichnet, dass die Konzentration von Taurolidin im Bereich von 2 bis 3 Gw.-% und/oder die Konzentration von Taurultam im Bereich von 3 bis 5 Gew.-% liegt.

8. Wässrige Lösung gemäss Anspruch 6, umfassend Taurolidin bei einer Konzentration im Bereich von 3 bis 4 Gew.-% und Glukose bei einer Konzentration im Bereich von 15 bis 25 Gew.-%.

9. Wässrige Lösung gemäss Anspruch 1, unfassend weiterhin einen Elektrolyt und/oder eine oder mehrere Aminosäure und/oder Spurenelemente und Vitamine.

10. Verfahren zur Herstellung einer wässrigen Lösung gemäss Anspruch 1, dadurch gekennzeichnet, dass das Taurolidin und/oder Taurultam in einer sterilen wässrigen Lösung des Polyols gelöst wird.

11. Verwendung von Taurolidin und/oder Taurultam zusammen mit einem parenteral annehmbaren Polyol bei der Herstellung einer sterilen wässrigen Lösung zur parenteralen Ernährung oder zur parenteralen Behandlung oder Prophylaxe von Infektionen.

12. Verfahren zum Löslichmachen von Taurolidin und/oder Taurultam in einer sterilen wässrigen Lösung zur parenteralen Verabreichung, bestehend in der Zugabe einer wirksamen Menge eines parenteral annehmbaren Polyols dazu.

13. Methode zum Löslichmachen von Taurolidin gemäss Anspruch 12, dadurch gekennzeichnet, dass das Polyol Glukose ist.

## Revendications

1. Solution aqueuse stérile pour administration parentérale contenant une concentration efficace en tant que bactéricide de taurolidine et/ou de taurultam ainsi qu'un polyol acceptable par voie parentérale.

2. Solution aqueuse selon la revendication 1, dans laquelle le polyol est le glycérol, un sucre ou un alcool-sucre.

3. Solution aqueuse selon la revendication 2, dans laquelle l'alcool-sucre est le sorbitol ou le xylitol.

4. Solution aqueuse selon la revendication 1, dans laquelle le polyol est le glucose.

5. Solution aqueuse selon la revendication 1, dans laquelle le polyol est le fructose.

6. Solution aqueuse selon la revendication 1, dans laquelle la concentration de taurolidine est dans la plage de 1 à 5% en poids et/ou la concentration de taurultam est dans la plage de 1 à 7,5% en poids.

7. Solution aqueuse selon la revendication 6, dans laquelle la concentration de taurolidine est dans la plage de 2 à 3% en poids et/ou la concentration de taurultam est dans la plage de 3 à 5%.

8. Solution aqueuse selon la revendication 6, comprenant la taurolidine a une concentration dans la plage de 3 à 4% en poids et le glucose à une concentration dans la plage de 15 à 25% en poids.

9. Solution aqueuse selon la revendication 1, comprenant en plus un électrolyte et/ou un ou plusieurs aminoacides et/ou des éléments marqueurs et des vitamines.

10. Procédé de préparation d'une solution aqueuse selon la revendication 1, dans lequel on dissout la taurolidine et/ou le taurultam dans une solution aqueuse stérile du polyol.

11. Utilisation de la taurolidine et/ou du taurultam avec un polyol acceptable par voie parentérale pour la fabrication d'une solution aqueuse stérile pour l'alimentation parentérale ou pour le traitement ou la prophylaxie des infections par voie parentérale.

12. Procédé de solubilisation de la taurolidine et/ou du taurultam dans une solution aqueuse stérile pour administration par voie parentérale, qui comprend l'addition à cette dernière d'une quantité efficace d'un polyol acceptable par voie parentérale.

13. Procédé de solubilisation selon la revendication 12, dans lequel le polyol est le glucose.